# EUROPÄISCHE PATENTSCHRIFT

(11) **EP 1 591 786 B1**
(45) Veröffentlichungstag und Bekanntmachung des Hinweises auf die Patenterteilung: **11.09.2013**
(21) Anmeldenummer: 05008438.3
(22) Anmeldetag: 19.04.2005
(51) Int. Cl.: G01N 33/03, G01N 25/04

(54) **Vorrichtung zum Erfassen von Kristallisations-Erstarrungskurven von Schokolade- und ähnlichen Fettmassen**
Apparatus for determining crystallization solidification curves of chocolate masses and similar fatty masses
Appareil pour déterminer des courves de solidification par cristallisation des masses de chocolat ou des masses graisseuses similaires

(30) Priorität: 29.04.2004 DE 102004021135
(43) Veröffentlichungstag der Anmeldung: 02.11.2005
(73) Patentinhaber: Sollich KG, 32105 Bad Salzuflen (DE)
(72) Erfinder: Sollich, Thomas, 32689 Kalletal (DE)
(74) Vertreter: Rehberg Hüppe + Partner

(56) Entgegenhaltungen:
- DE-A1- 4 209 073
- DE-C1- 3 714 951

## Beschreibung

Die Erfindung betrifft eine Vorrichtung zum Erfassen von Kristallisations-Erstarrungskurven von Schokolade- und ähnlichen Fettmassen, mit einem Gehäuse, einer darin gebildeten Entnahmestelle für flüssige Schokolade, mit einer an die Entnahmestelle angeschlossenen und eine gekühlte Wandung aufweisenden Messkammer, in der die flüssige Schokolademasse zur Erstarrung gebracht wird, mit einem im Gehäuse vorgesehenen bewegbaren Kolben, mit einem im Gehäuse vorgesehenen Temperaturfühler und mit einer Einrichtung zum Entfernen der erstarrten Probe aus der Messkammer. Solche Vorrichtungen werden branchenüblich auch als Tempermeter bezeichnet. Sie dienen dazu, den Temperiergrad einer flüssigen Schokolade oder einer ähnlichen Fettmasse festzustellen. Solche Tempermeter werden oft automatisch betrieben, indem in einstellbaren zeitlichen Abständen Proben flüssiger Schokolade an einer Entnahmestelle entnommen und zur Erstarrung gebracht werden. Ein Temperaturfühler etwa im Zentrum dieser Probe misst den Verlauf der Temperatur und gibt diese an eine Auswerteeinrichtung weiter, mit deren Hilfe der Temperaturverlauf über der Zeit festgehalten, insbesondere dargestellt wird. Der Verlauf dieser Temperaturkurve gibt dem Fachmann Aufschluss über den Temperiergrad oder Temperierindex und damit über die Qualität der vorkristallisierten flüssigen Schokolade. Solche Tempermeter können auch im Rahmen einer Steuer- oder Regeleinrichtung eingesetzt werden, um die erforderlichen Anpassungen bei vorliegender Über- oder Untertemperierung an die Einstellbedingungen beispielsweise einer Temperiermaschine vorzunehmen, damit letztendlich auf das gewünschte Arbeitsergebnis abgestimmte ideal temperierte Schokolade erhalten und verarbeitet werden kann.

### STAND DER TECHNIK

Eine Vorrichtung der eingangs beschriebenen Art ist aus der DE 37 14 951 C1 bekannt. Es ist eine Messkammer vorgesehen, die von einer gekühlten Wandung umschlossen wird. Die Messkammer ist an einer Entnahmestelle für flüssige vorkristallisierte Schokolade angeschlossen, beispielsweise an einen Rohrleitungsabschnitt, aus dem die flüssige Schokolade für die Probe entnommen wird. Die Messkammer ragt teilweise in den Strömungsquerschnitt der Rohrleitung ein. Die Messkammer selbst besitzt zylindrischen Querschnitt und ist damit auf einen in der Messkammer verfahrbar angeordneten zylindrischen Kolben abgestimmt. Der Kolben trägt einen Temperaturfühler, der die vordere Wandung des Kolbens überragt und in der zurückgezogenen Stellung des Kolbens in die Messkammer einragt und dort die Temperatur der erstarrenden Probe verfolgt und aufnimmt. Die Messkammer wird damit von einer Kolben/Zylinder-Einheit gebildet, die mit ihrer offenen Stirnseite in die zu erfassende Schokolademasse eintauchend angeordnet ist. Es ist ein Antrieb für den Hub des Kolbens vorgesehen, der etwa auf die Länge der Messkammer bemessen ist. Weiterhin ist eine Einrichtung zum Entfernen der erstarrten Probe von dem Kolben und dem Temperaturfühler vorgesehen. Diese Einrichtung besteht aus einer mechanischen Zerkleinerungseinrichtung, insbesondere einer Fräs- und Schneideinrichtung, deren Achse fluchtend zu der Achse der Messkammer angeordnet ist. Die Fräs- oder Schneideinrichtung besitzt eine über einen Motor angetriebene Welle und einen Fräskopf, der in den Strömungsquerschnitt der Messstelle von außen her einragt und den Strömungsquerschnitt teilweise versperrt. Die einragende Welle muss abgedichtet werden.

Die bekannte Vorrichtung wird wie folgt betrieben. Durch einen Rückhub des Kolbens in der Messkammer wird flüssige Schokolademasse aus der Entnahmestelle angesaugt und in die Messkammer überführt. Durch den Einfluss der gekühlten Wandung der Messkammer läuft ein Erstarrungsprozess der flüssigen Probe ab, bis das gesamte Material der Probe verfestigt ist. Um die nächste Probe an flüssiger Schokolade in die Messkammer ansaugen zu können, muss zunächst die erstarrte Probe aus der Messkammer entfernt werden. Dies geschieht durch die mechanische Zerkleinerungseinrichtung, mit der die erstarrte Probe aufgefräst wird. Die dabei entstehenden festen Partikel werden über den Fräsvorgang hinweg der flüssigen Schokolademasse, die durch die Entnahmestelle strömt, hinzugefügt, abgefördert und aufgeschmolzen. Dieser Zerkleinerungs- und Auflösevorgang der erstarrten Probe dauert einige Minuten. Während dieser Zeit ist die Messkammer blockiert, d. h. es kann keine neue Probe in die Messkammer überführt werden. Vielmehr muss der komplette Ablauf des Zerkleinerungs- und Auflösevorgangs der erstarrten Probe abgewartet werden, bevor eine neue Probe gezogen werden kann. Die Zeitdauer für die Untersuchung einer Probe ist damit relativ lang, so dass auf diese Weise nur Proben in entsprechenden zeitlichen Abständen genommen werden können. Oft erfordert aber ein Steuer- oder Regelsystem eine zeitlich schnellere Aufeinanderfolge verschiedener Proben.

Die bekannte Vorrichtung besitzt den weiteren Nachteil, dass die mechanische Zerkleinerungseinrichtung einen eigenen Antrieb erfordert, der außerhalb der Entnahmestelle vorgesehen ist und rotierend angetriebene Elemente aufweist. Damit ist z. B. der Fräskopf der mechanischen Zerkleinerungseinrichtung unzugänglich für Wartungs- und Kontrollzwecke im Innern der Entnahmestelle angeordnet. Außerdem erfordert die Durchführung der Antriebswelle durch die Wandung der Entnahmestelle, beispielsweise in Form einer Rohrleitung oder eines Rohrleitungsabschnittes, eine Dichtung. Diese Dichtung stellt ein Verschleißteil dar und muss regelmäßig gewechselt werden, damit ein Schokoladenaustritt verhindert wird.

Ein weiterer Nachteil besteht darin, dass eine exakte Steuerung des Antriebs für den Kolben in der Messkammer und für die mechanische Zerkleinerungseinrichtung erforderlich ist. So darf der Vorwärtshub des Kolbens mit der erstarrten Probe nicht schneller erfolgen, als die Zerkleinerungseinheit die erstarrte Probe abfräsen kann.

### AUFGABE DER ERFINDUNG

Der Erfindung liegt die Aufgabe zugrunde, eine Vorrichtung der eingangs beschriebenen Art bereitzustellen, mit der insbesondere automatisch aufeinander folgende Proben in vergleichsweise kürzeren zeitlichen Abständen gezogen und erfasst werden können.

### LÖSUNG

Die Aufgabe der Erfindung wird erfindungsgemäß mit den Merkmalen des unabhängigen Patentanspruchs 1 gelöst.

### BESCHREIBUNG DER ERFINDUNG

Die Erfindung geht von dem Gedanken aus, eine separate Aufschmelzkammer zum Aufschmelzen der erstarrten Probe vorzusehen. Die Aufschmelzkammer und die Messkammer sind damit örtlich getrennt voneinander vorgesehen, vorzugsweise in einem Abstand, der mindestens dem Durchmesser des Strömungsquerschnittes an der Entnahmestelle entspricht. Lediglich die Anordnung der Aufschmelzkammer und der Messkammer wird so aufeinander abgestimmt, dass deren Achsen fluchtend oder parallel zueinander vorgesehen sind. In der Regel besitzen sowohl die Messkammer wie auch die Aufschmelzkammer kreisförmigen oder zumindest teilzylindrischen Querschnitt.

In einer ersten Ausführungsform, die sich durch fluchtende Achsen der Messkammer und der Aufschmelzkammer kennzeichnet wird der geradlinig hin- und hergehende Hub des Kolbens im Vergleich zu dem Hub des Kolbens der bekannten Vorrichtung verlängert, damit eine erstarrte Probe aus der Messkammer in die Aufschmelzkammer überführt werden kann und der Strömungsquerschnitt der Entnahmestelle während des Rückhubes des Kolbens in die Messkammer sofort wieder frei wird, damit während und mit diesem Rückhub, ohne dass die erstarrte Probe in der Aufschmelzkammer bereits aufgeschmolzen ist, eine nachfolgende Probe flüssiger Schokoladenmasse in die Messkammer eingesaugt werden kann. Während des zeitlichen Ablaufs der Erstarrung der nachfolgenden Probe wird die vorangehende erstarrte Probe in der Aufschmelzkammer aufgeschmolzen. Dies führt zu einer erheblichen Reduzierung des zeitlichen Taktes, mit dem aufeinander folgend Proben untersucht werden können, weil sich der Erstarrungsvorgang der Probe in der Messkammer und der Aufschmelzvorgang in der Aufschmelzkammer zeitlich überlagern.

In oder an der Aufschmelzkammer ist eine Einrichtung zum Festhalten der erstarrten Probe vorgesehen, die bei oder während des Einschiebens der erstarrten Probe in die Aufschmelzkammer wirksam wird und die erstarrte Probe in der Aufschmelzkammer festhält, während sich der Kolben von der erstarrten Probe zu Beginn seines Rückhubes löst.

In einer zweiten Ausführungsform ist der Kolben als Drehkolben ausgebildet und weist mindestens drei Kammern auf, die je nach Drehstellung des Drehkolbens abwechselnd die Messkammer, die Aufschmelzkammer und eine an die Entnahmestelle angeschlossene Kammer bilden. Der Drehkolben ist in einem Gehäuse getaktet rotierend angetrieben, wobei die Kammern an dem Drehkolben als teilzylindrische Längsnuten ausgebildet sind. In der Übernahmestellung schließt eine Kammer an die Entnahmestelle an. In einer zweiten Stellung wird die Kammer samt aus der Entnahmestelle entnommener flüssiger Schokolademasse in eine Position gedreht, in der die Kammer die Funktion der Messkammer erbringt. Ein Temperaturfühler ist im Gehäuse gelagert und in die Kammer in dieser Stellung einfahrbar und ausfahrbar. Im Gehäuse ist in diesem Bereich eine Kühleinrichtung vorgesehen, mit der die Schokolademasse zur Erstarrung gebracht wird. Dabei wird die Erstarrungskurve aufgezeichnet. Separat zu der Messkammer ist die Aufschmelzkammer vorgesehen bzw. gebildet, in der über eine im Gehäuse vorgesehene Heizeinrichtung die Probe wieder aufgeschmolzen wird. Die Kammer des Drehkolbens dreht in diese Position weiter. In der dritten und letzten Stellung dreht die Kammer des Drehkolbens schließlich wiederum weiter und bekommt Anschluss an die Entnahmestelle, sodass die in der Aufschmelzkammer wieder aufgeschmolzene Probe wieder in den Strom der Schokolademasse abgeführt wird. Der besondere Vorteil dieser Ausführungsform besteht in einer weiter verkürzten Zykluszeit, d. h. es können mehr Proben pro Zeiteinheit entnommen und somit die Erstarrungskurven in kürzeren zeitlichen Abständen aufgezeichnet werden. Dies führt zu einer noch genaueren Prozesskontrolle.

Die neue Vorrichtung vermeidet durch die separate Aufschmelzkammer auch alle Nachteile, die mit einer mechanischen Zerkleinerung der erstarrten Probe verbunden sind. Insbesondere wird die Rückführung von festen Partikeln, wie sie beim Fräsvorgang entstehen, in den Strömungsweg der flüssigen vorkristallisierten Schokolade vermieden. Der Aufschmelzvorgang kann schonend und gleichmäßig durchgeführt werden. Es müssen keine besonders großen Temperaturdifferenzen angewendet werden, so dass die Schokolademasse nicht geschädigt wird. Dies ist deshalb möglich, weil für den Aufschmelzvorgang grundsätzlich die Zeitspanne oder etwa die Zeitspanne zur Verfügung steht, die auch der Erstarrungsvorgang der nachfolgenden Probe erfordert.

Die Vorrichtung kann als Entnahmestelle den Strömungsquerschnitt in einem Rohrleitungsabschnitt nutzen. Die Vorrichtung ist damit in bzw. an diesem Rohrleitungsabschnitt angeordnet. Ein solcher Rohrleitungsabschnitt lässt sich in einfacher Weise in jeder Rohrleitung einbauen, durch die flüssige Schokolademasse beispielsweise aus einer Temperiermaschine zu einer Verarbeitungsmaschine geleitet wird.

Es empfiehlt sich bei einer ersten Ausführungsform die Messkammer einerseits und die Aufschmelzkammer andererseits etwa symmetrisch zu der Achse des Rohrleitungsabschnitts gegenüberliegend anzuordnen. Dabei stehen die gemeinsame Achse der Messkammer und der Aufschmelzkammer senkrecht auf der Achse des Rohrleitungsabschnitts. Da die Aufschmelzkammer keine angetriebenen bewegten Elemente aufweist, entfällt vorteilhaft auch eine Abdichtung der im Stand der Technik erforderlichen Antriebswelle für die mechanische Zerkleinerungseinrichtung. Durch den Entfall der Dichtung wird die Vorrichtung erheblich betriebssicherer und erfordert eine reduzierte Wartung.

Die Messkammer und/oder die Aufschmelzkammer sind vorzugsweise etwa zylindrisch ausgebildet, d. h. sie besitzen einen kreisförmigen Querschnitt. Es versteht sich, dass der Querschnitt bzw. Durchmesser der Aufschmelzkammer nicht kleiner bemessen sein darf als der Durchmesser der Messkammer. Es ist aber durchaus sinnvoll, dass sich sowohl die Messkammer wie auch die Aufschmelzkammer zumindest im Wesentlichen außerhalb des Strömungsquerschnittes der Entnahmestelle befinden, damit dieser Strömungsquerschnitt durch die Anordnung der beiden Kammern nicht wesentlich beeinträchtigt wird. Allerdings ist es erforderlich, dafür zu sorgen, dass sich die aus der Messkammer ausgeschobene und in die Aufschmelzkammer einzuschiebende erstarrte Probe nicht vorzeitig von dem Kolben löst und dann von der im Strömungsquerschnitt der Entnahmestelle strömenden Schokolade mitgenommen wird. Dies könnte zu Störungen des Betriebsablaufs führen. Um dieser Gefahr entgegenzuwirken, ist im Strömungsquerschnitt der Entnahmestelle ein Führungskäfig für die Überführung der erstarrten Probe aus der Messkammer in die Schmelzkammer vorgesehen. Dieser Führungskäfig leitet und führt die erstarrte Probe auf ihrem Weg aus der Messkammer in die Aufschmelzkammer. Sie verhindert selbst bei einem vorzeitigen Lösen der erstarrten Probe von der Oberfläche des Kolbens eine Mitnahme durch die flüssige Schokolade, die durch die Entnahmestelle kontinuierlich strömt. Damit ist sichergestellt, dass die erstarrte Probe sicher und reproduzierbar in die Aufschmelzkammer überführt und dort dann nachfolgend aufgeschmolzen wird. Der Führungskäfig ist zweckmäßig als durchbrochener Hülsenabschnitt ausgebildet, dessen Achse fluchtend zu der gemeinsamen Achse der Messkammer und der Aufschmelzkammer angeordnet ist. Der Hülsenabschnitt sollte großflächige Durchbrechungen aufweisen, um die Durchströmung der Entnahmestelle mit flüssiger Schokolade möglichst wenig zu beeinträchtigen. Statt des durchbrochenen Hülsenabschnittes kann auch eine käfigartige Konstruktion aus Drahtabschnitten eingesetzt werden, die die beschriebene Führungs- und Leitungsfunktion erfüllt.

In der zweiten Ausführungsform, bei der der Kolben als Drehkolben ausgebildet ist, wechseln die am Drehkolben vorgesehenen Kammern ihre Funktionen, je nach der relativen Stellung im Gehäuse. Dies hat den Vorteil, dass ein Führungskäfig entbehrlich wird. Der Querschnitt der Rohrleitung bleibt in jeder Stillstandsstellung des Drehkolbens auch an der Entnahmestelle völlig frei und wird damit auch nicht teilversperrt.

Die Messkammer ist mit einer Kühleinrichtung und die Aufschmelzkammer mit einer Heizeinrichtung versehen. Dem Fachmann bieten sich hier verschiedene Realisierungsmöglichkeiten. Die Kühlung kann durch Kühlwasser oder thermoelektrisch beispielsweise durch ein Peltier-Element, erfolgen. Zu diesem Zweck kann die Wandung der Messkammer doppelwandig ausgebildet und an einen Kreislauf für Kühlwasser angeschlossen sein. Die Temperatur des Kühlwassers kann über die Zeit konstant gehalten werden, d. h. es ist vorteilhaft nicht erforderlich, die Temperatur des Kühlwassers in Intervallen zu ändern. Der Antrieb des Kolbens genügt, um die erstarrte Probe auch von der Innenwandung der Messkammer zu lösen. Der entsprechende Vorteil einer zeitlich konstanten Temperatur ergibt sich auch für die Aufschmelzkammer bzw. den Anschluss einer doppelten Wandung an konstant temperiertes Warmwasser. Damit entfällt vorteilhaft das periodische Aufheizen bzw. Abkühlen metallischer Wandungen.

Die in die Aufschmelzkammer eingeführte erstarrte Probe muss während des Rückhubes des Kolbens in der Aufschmelzkammer festgehalten werden. Hierzu ist es zweckmäßig, dass die Einrichtung zum Festhalten der erstarrten Probe während des Rückhubes des Kolbens eine Aufnahmehülse aufweist, in der oder an der Rückhalteelemente für die eingeschobene erstarrte Probe vorgesehen sind. Diese Rückhalteelemente müssen die erstarrte Probe verlässlich festhalten und ein Lösen des Kolbens von der rückwärtigen Stirnfläche der erstarrten Probe bei beginnendem Rückhub des Kolbens ermöglichen. Als Rückhalteelemente kann ein Kugelventil, welches der Aufnahmehülse zugeordnet ist, gebogene Klingen, Widerhaken oder dergleichen dienen, die die eingeschobene erstarrte Probe relativ zur Aufnahmehülse fixieren.

### KURZBESCHREIBUNG DER FIGUREN

Im Folgenden wird die Erfindung anhand eines in den Figuren dargestellten bevorzugten Ausführungsbeispiels weiter erläutert und beschrieben.
- **Fig. 1**: zeigt einen Querschnitt durch eine erste Ausführungsform der Vorrichtung, und zwar in einer mittleren Stellung des Kolbens zu Beginn eines Ansaughubes des Kolbens in der Messkammer.
- **Fig. 2**: zeigt eine Stellung, in der der Kolben in der Messkammer seinen Rückhub vollendet hat und der Erstarrungsvorgang der eingesaugten Probe abläuft.
- **Fig. 3**: zeigt einen Querschnitt durch die Ausführungsform der Fig. 1 und 2 kurz vor der vollständigen Überführung der erstarrten Probe in die Aufschmelzkammer.
- **Fig. 4**: zeigt schließlich einen Schnitt etwa gemäß der Schnittangabe IV-IV in Fig. 1 mit der Darstellung der Rückhalteelemente.
- **Fig. 5**: zeigt einen Schnitt durch eine zweite Ausführungsform der Vorrichtung.
- **Fig. 6**: zeigt einen Schnitt ähnlich Fig. 5 um eine halbe Teilung weitergedreht.
- **Fig. 7**: zeigt einen Schnitt ähnlich Fig. 5 um eine ganze Teilung weitergedreht.
- **Fig. 8**: zeigt schließlich einen Vertikalschnitt etwa gemäß der Schnittangabe VIII-VIII in Fig. 5.

### FIGURENBESCHREIBUNG

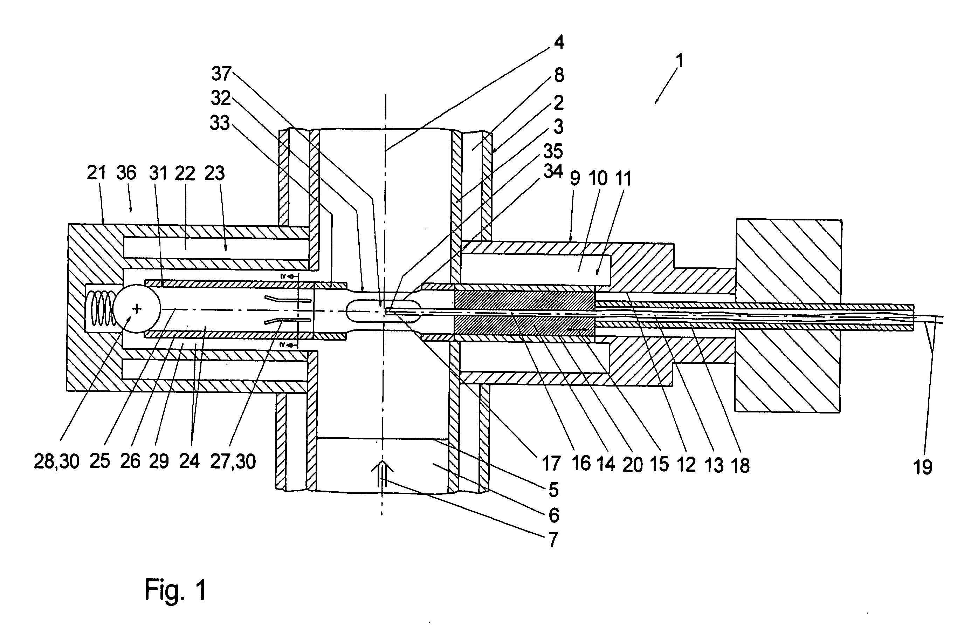

Fig. 1 zeigt eine bevorzugte Ausführungsform der Vorrichtung 1. Die Vorrichtung 1 weist ein Gehäuse 2 auf, welches als Rohrleitungsabschnitt 3 ausgebildet ist. Das Gehäuse 2 kann auch eine andere Form aufweisen. Ein Rohrleitungsabschnitt 3 wird jedoch bevorzugt, weil sich so die Vorrichtung in sehr einfacher Weise über entsprechende Flanschverbindungen an dem Rohrleitungsabschnitt in Leitungen einbauen lässt. Das Gehäuse 2 bzw. der Rohrleitungsabschnitt 3 weist eine Achse 4 auf. Im Innern des Gehäuses 2 bzw. des Rohrleitungsabschnittes 3 ist so ein Strömungsquerschnitt 5 gebildet, der hier kreisförmig ausgebildet ist und durch den flüssige Schokolademasse 6 in Richtung des Pfeils 7 von unten nach oben strömt. In der Regel handelt es sich um eine kontinuierliche Strömung der Schokolademasse 6, angetrieben durch eine nicht dargestellte Pumpe. Das Gehäuse 2 der Vorrichtung 1 ist in aller Regel doppelwandig ausgebildet. Ein auf diese Weise gebildeter Raum 8 ist an einen Kreislauf mit temperiertem Wasser angeschlossen, der als Schutzheizung fungiert und verhindert, dass die flüssige Schokolademasse während ihrer Durchströmung über die Länge des Rohrleitungsabschnittes 3 einer Temperaturänderung unterliegt.

Seitlich am Gehäuse 2 bzw. an der Rohrleitung 3 ist eine Messkammer 9 angeordnet, die ebenfalls doppelwandig ausgebildet ist. Der gebildete Zwischenraum ist an einen Kreislauf 10 für Kühlwasser angeschlossen. Der Kreislauf 10 des Kühlwassers wird mit einstellbarer, aber konstanter Temperatur betrieben. Auf diese Weise wird eine Kühleinrichtung 11 für die Messkammer 9 geschaffen.

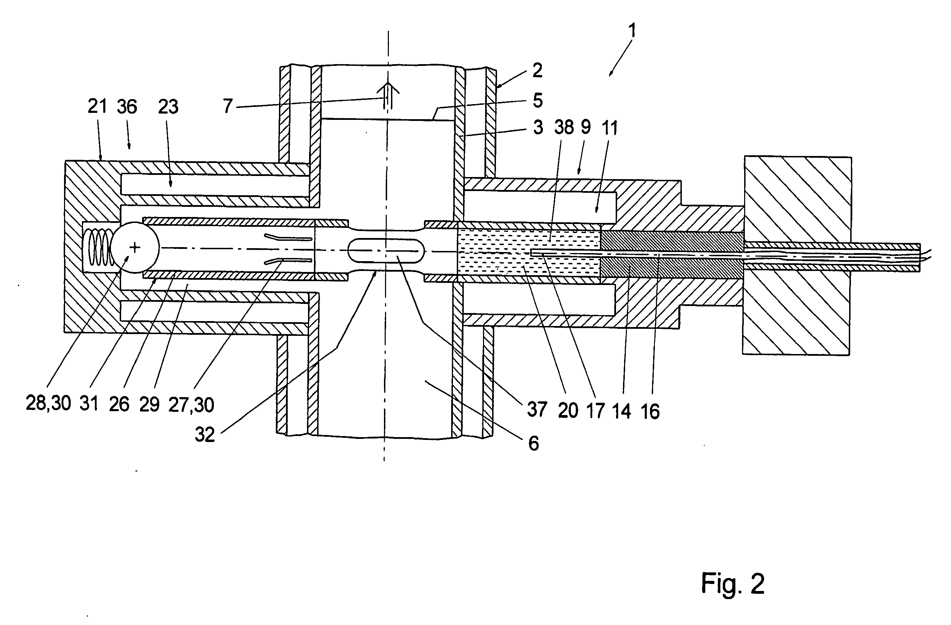

Im Innern der Messkammer 9 wird ein hohler Zylinder 12 gebildet, der Anschluss an den Strömungsquerschnitt 5 des Gehäuses 2 aufweist. Der Zylinder 12 besitzt eine Achse 13, die senkrecht zu der Achse 4 des Strömungsquerschnittes 5 angeordnet ist. In dem Zylinder 12 ist ein Kolben 14 vorgesehen, der über einen nicht dargestellten Antrieb in Richtung der Achse 13 und damit des Doppelpfeils 15, also mit Vor- und Rückhub, verfahrbar ist. Der Kolben 14 trägt einen Temperaturfühler 16, der in dem Kolben 14 zentrisch angeordnet ist und dessen vorderes Ende 17 frei ausragt. Der Kolben 14 weist eine Kolbenstange 18 auf, die einerseits dem Antrieb des Kolbens 16 dient und durch die andererseits Anschlussleitungen 19 des Temperaturfühlers 16 nach außen zu einer nicht dargestellten Auswerte- und Aufnahmeeinrichtung geführt sind. Ein vorderer Teil des Zylinders 12, im Anschluss an den Strömungsquerschnitt 5, bildet einen Messraum 20. Der Messraum 20 befindet sich etwa dort, wo gemäß Fig. 1 der verfahrbare Kolben 14 dargestellt ist. Wenn der Kolben 14 an seinen rückwärtigen Todpunkt gelangt, wie dies in Fig. 2 dargestellt ist, wird der Messraum 20 voll sichtbar.

Auf der der Messkammer 9 gegenüberliegenden Seite des Gehäuses 2 bzw. des Rohrleitungsabschnittes 3 ist eine Aufschmelzkammer 21 angeordnet. Auch die Aufschmelzkammer 21 ist doppelwandig ausgebildet. Der gebildete Zwischenraum ist an einen Kreislauf 22 für Warmwasser angeschlossen. Auf diese Weise wird eine Heizeinrichtung 23 gebildet. Auch andere Ausführungsmöglichkeiten der Heizeinrichtung 23 sind denkbar. Die Heizeinrichtung 23 könnte auch elektrisch ausgebildet sein. Der Kreislauf 22 wird ebenfalls mit Wasser betrieben, hier mit Warmwasser. Die Temperatur ist einstellbar und kann über die Probezeit konstant bleiben. Die gewählte Temperatur muss ausreichend sein, eine erstarrte Probe aus Schokolademasse aufschmelzen zu können. Andererseits muss die Temperatur hinreichend niedrig gewählt werden, damit die Eigenschaften der durch den Strömungsquerschnitt 5 strömenden Schokolademasse 6 nicht negativ beeinflusst werden. Im Innern der Aufschmelzkammer 21 ist ein Schmelzraum 24 gebildet. Der Schmelzraum 24 kann ebenso wie der Messraum 20 kreisrunden Querschnitt aufweisen. Er besitzt zweckmäßig einen vergleichsweise geringfügig größeren Durchmesser als der Messraum 20 bzw. ein zwischengeschalteter Führungskäfig 32, damit eine erstarrte Probe aus dem Messraum in den Schmelzraum überführt werden kann. Berücksichtigt man, dass eine Schokolademasse beim Erstarren schrumpft, so können der Messraum 20, der Führungskäfig 32 und eine Aufnahmehülse 26 im Schmelzraum 24, beispielsweise in Form eines durchgehenden Rohrabschnitts, übereinstimmenden Innendurchmesser aufweisen. Die Aufschmelzkammer 21 bzw. der Schmelzraum 24 weisen eine Achse 25 auf, die fluchtend zu der Achse 13 des Messraums 20 angeordnet ist. In dem Schmelzraum 24 ist die Aufnahmehülse 26 ortsfest gelagert. In Zuordnung zu der ortsfesten Aufnahmehülse 26 sind gebogene Klingen 27 angeordnet. Es können auch widerhakenähnliche Elemente vorgesehen sein. Alternativ oder zusätzlich kann die Aufnahmehülse 26 an ihrem dem Strömungsquerschnitt 5 abgekehrten Ende ein Kugelventil 28 aufweisen, welches wie dargestellt eine Kugel und eine Feder aufweist. Die Aufnahmehülse 26 kann mit nicht dargestellten Durchbrechungen versehen sein. Der Außendurchmesser der Aufnahmehülse 26 ist kleiner als der Innendurchmesser des Schmelzraums 24, um einen Überströmkanal 29 für die Rückströmung aufgeschmolzener Schokolademasse in den Strömungsquerschnitt 5 zu schaffen. Die Klingen 27, Widerhaken und/oder das Kugelventil 28 bilden Rückhalteelemente 30. Zusammen mit der Aufnahmehülse 26 wird damit eine Einrichtung 31 zum Festhalten oder Rückhalten einer eingeschobenen erstarrten Probe 38 in die Aufschmelzkammer 21 geschaffen. Die Elemente der Einrichtung 31 zum Festhalten einer erstarrten Probe sind beispielhaft zu verstehen. Dem Fachmann bieten sich auch andere Realisierungsmöglichkeiten.

Zwischen der Messkammer 9 und der Aufschmelzkammer 21 ist der Führungskäfig 32 ortsfest angeordnet. Der Führungskäfig 32 weist einen Hülsenabschnitt 33 auf, der mit großformatigen Durchbrechungen 34 versehen ist. Der Führungskäfig 32 dient dazu, eine erstarrte Probe während ihrer Überführung aus dem Messraum 20 in den Schmelzraum 24 zu leiten und zu führen und damit ein Aufschwimmen im Strömungsquerschnitt 5 in Richtung des Pfeils 7 zu verhindern. Der Führungskäfig 32 könnte auch als leichte Drahtkonstruktion ausgebildet sein. Er ist in der Regel mit der Aufnahmehülse 26 verbunden bzw. an dieser abgestützt. Er könnte auch einstückig mit der Aufnahmehülse 26 gestaltet werden. Der Führungskäfig 32 bzw. der Hülsenabschnitt 33 weist eine Achse 35 auf. Die Achse 35 fluchtet mit den Achsen 13 und 25, so dass auf diese Art und Weise eine gemeinsame Achse gebildet wird.

Der Hub des Kolbens 14 ist so bemessen, dass er bis zum Ende des Führungskäfigs 32 bzw. zum Anfang der Aufnahmehülse 26 der Aufschmelzkammer 21 bewegt werden kann, um eine erstarrte Probe aus dem Messraum 20 der Messkammer 9 in die Aufnahmehülse 26 der Aufschmelzkammer 21 zu überführen. Der Kolben 14 mit seiner Kolbenstange 18 und dem zugehörigen Antrieb bilden im Wesentlichen die beweglichen Teile der Vorrichtung 1. Die Aufschmelzkammer 21 mit dem Schmelzraum 24, den Rückhalteelementen 30 und der Heizeinrichtung 23 bilden zusammen oder mit zusätzlichen Elementen eine Einrichtung 36 zum Entfernen einer erstarrten Probe 38 von dem Kolben 14 und dem Temperaturfühler 16 sowie zum Aufschmelzen und Rückleiten dieser aufgeschmolzenen Schokolademasse in den Strömungsquerschnitt 5.

Die Vorrichtung 1 wird vorzugsweise kontinuierlich betrieben, d. h. in festgelegten Intervallen werden Proben der Schokolademasse 6 aus dem Strömungsquerschnitt 5 genommen, zur Erstarrung gebracht und wieder aufgeschmolzen. Ein solcher Zyklus läuft wie folgt ab:

Im Strömungsquerschnitt 5 des Gehäuses 2 ist eine Entnahmestelle 37 gebildet, die immer von flüssiger Schokolademasse 6 durchströmt wird. Diese Entnahmestelle 37 kann im Bereich der großflächigen Durchbrechungen 34 des Führungskäfigs 32 vorgesehen sein oder aber auch in dem dem Messraum 20 der Messkammer 9 zugekehrten Wandbereich des Gehäuses 2, falls sich der Führungskäfig 32 an der Wandung des Rohrabschnittes 3 an dem Messraum 20 nicht abstützt, also etwas kürzer gestaltet ist. Auf jeden Fall muss der Messraum 20 immer an den Strömungsquerschnitt 5 angeschlossen sein, damit bei einem Rückhub des Kolbens 14 beim Übergang aus der Stellung der Fig. 1 in die Stellung gemäß Fig. 2 ein Saughub der Messkammer 9 realisiert wird, durch welchen der Messraum 20 mit frischer flüssiger Schokolademasse 6 gefüllt wird, die dann durch Einwirkung der Kühleinrichtung 11 zur Erstarrung gebracht wird. Mit diesem Vorgang bildet sich in dem Messraum 20 die erstarrte Probe 38, also ein zylindrischer Pfropfen fester Schokolademasse. Dieser Erstarrungsvorgang wird von dem Temperaturfühler 16 erfasst, dessen vorderes Ende 17 an repräsentativer Stelle des Messraums 20 bzw. der erstarrten Probe 38 angeordnet ist. Der Temperaturverlauf der Probe 38 wird in bekannter Weise aufgezeichnet und ausgewertet.

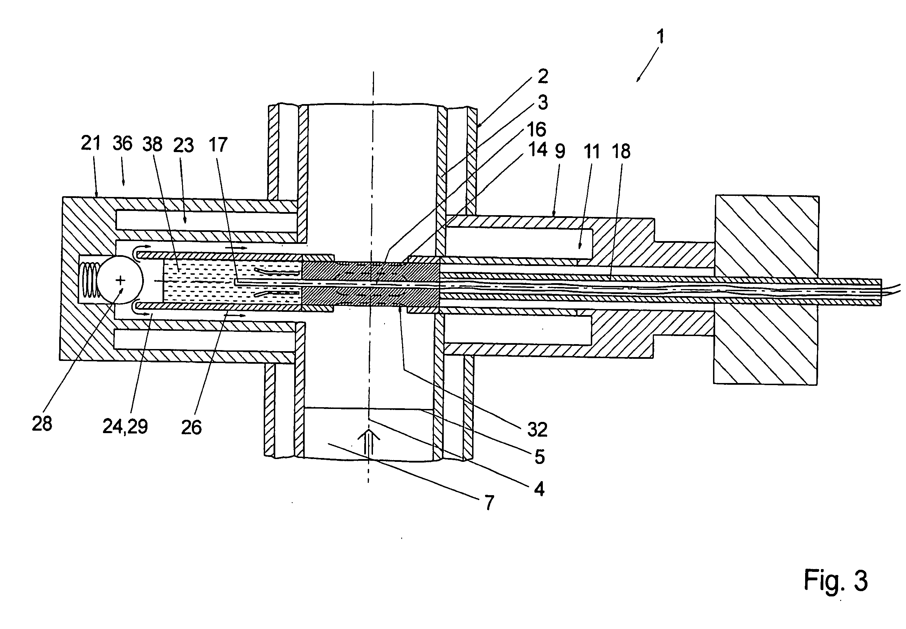

Nach der vollständigen Erstarrung der Probe 38 wird der Antrieb des Kolbens 14 in Gang gesetzt, so dass dieser seinen Vorwärtshub ausführt. Diese Vorgänge sind am Übergang aus der Stellung gemäß Fig. 2 in die Stellung gemäß Fig. 3 verdeutlicht. Zu Beginn des Vorwärtshubes des Kolbens 14 löst sich die Umfangsfläche der Probe 38 von der Innenwandung des Messraums 20, während die dem Kolben 14 zugekehrte Stirnwandung der Probe 38 an dem Kolben 14 haftet. Während des Vorwärtshubes gelangt die erstarrte Probe 38 zunächst in den Bereich des Führungskäfigs 32, durch dessen Hohlraum die Probe 38 geführt und geleitet wird. Der Führungskäfig 32 verhindert, dass sich die Probe 38 von der Stirnfläche des Kolbens 14 löst. Wenn sich die Probe 38 im Bereich des Strömungsquerschnittes 5 lösen würde, könnte sie von der gemäß Pfeil 7 strömenden Schokolademasse 6 mitgenommen werden und insoweit nicht in die Aufschmelzkammer 21 gelangen. Dies muss verhindert werden, und zwar entweder durch die Anordnung eines Führungskäfigs 32 oder durch entsprechende Vergrößerung der Kontaktfläche im stirnseitigen Bereich zwischen Kolben 14 und Probe 38. Der Führungskäfig 32 mit seinen großflächigen Durchbrechungen 34 stört jedoch die Strömung im Strömungsquerschnitt 5 nicht nennenswert, so dass seine Anordnung sinnvoll ist. Der Führungskäfig 32 ist auf die Aufnahmehülse 26 der Aufschmelzkammer 21 abgestimmt und so dimensioniert, dass bei fortgesetztem Vorwärtshub des Kolbens 14 die erstarrte Probe 38 in die Aufnahmehülse 26 gelangt (Fig. 3). Die dabei in der Aufnahmehülse 26 vorhandene schmelzflüssige Schokolademasse wird durch Öffnen des Kugelventils 38 (siehe Pfeildarstellung in Fig. 3) in den Überströmkanal 29 und von dort in den Strömungsquerschnitt 5 verschoben. Die Rückhalteelemente 30 bzw. die Einrichtung 31 zum Festhalten der erstarrten Probe 38 in der Aufnahmehülse 26 bzw. der Aufschmelzkammer 21 treten in Funktion. Dabei kann durch Schließen des Kugelventils 28 nach Erreichen des vorderen Todpunkts des Hubes des Kolbens 14 bereits ein gewisses Festhalten der erstarrten Probe 38 erfolgen, wenn der Kolben 14 seinen Rückhub beginnt. Diese Wirkung ist besonders groß, wenn die Aufnahmehülse 26 keine Durchbrechungen an ihrer zylindrischen Wand aufweist. Alternativ oder zusätzlich können die gebogenen Klingen 27 oder andere widerhakenförmige Elemente im Bereich der Aufnahmehülse 26 die Rückhaltung der in die Aufnahmehülse 26 eingeschobenen erstarrten Probe begünstigen und damit ein Lösen der Stirnfläche des Kolbens 14 von der erstarrten Probe 38 möglich machen. Die erstarrte Probe 38 befindet sich damit in der Aufschmelzkammer und verbleibt dort, während der Kolben 14 seinen Rückhub durchläuft. Sobald er die Stellung gemäß Fig. 1 dabei durchläuft, beginnt bereits wieder ein neuer Saughub und das Einsaugen der nachfolgenden Probe flüssiger Schokolademasse 6 in den Messraum 20. Während diese Messung und Aufnahme der Erstarrungskurve stattfindet, wird die feste Probe 38 des vergangenen Zyklus in der Aufschmelzkammer 21 aufgeschmolzen, also in den schmelzflüssigen Zustand überführt. Während der Überführung der erstarrten Probe 38 des zweiten Zyklus wird dann die schmelzflüssig erwärmte Schokolademasse der vorangehenden Probe während der Überführung verdrängt und in den Strömungsquerschnitt 5 zurückgeleitet. Damit wird erkennbar, dass die Erstarrung einer nachfolgenden Probe und das Aufschmelzen der vorangehenden Probe zeitüberdeckend durchgeführt werden können. Diese Überdeckung kann ganz oder teilweise genutzt werden. Auf jeden Fall wird die Taktzeit bei einer solchen automatischen Überwachung einer fließenden Schokolademasse erheblich kürzer.

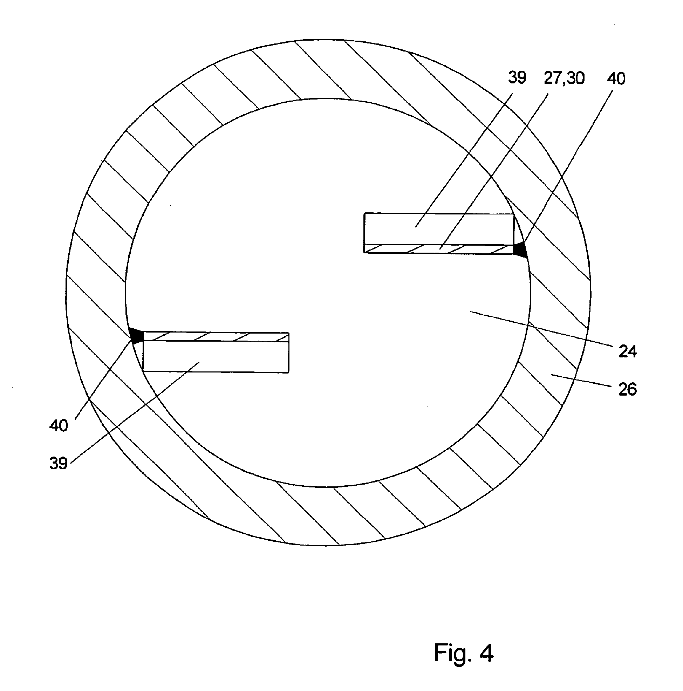

In Fig. 4 sind Einzelheiten der Einrichtung 31 zum Festhalten einer erstarrten und in die Aufnahmehülse 26 überführten Probe 38 dargestellt, allerdings ohne Darstellung der Probe selbst. Am inneren Umfang der Aufnahmehülse 26 sind in der dargestellten Relativlage zwei Klingen 27 vorgesehen, die, wie auch aus Fig. 1 erkennbar, abgebogene federnde Enden 39 aufweisen. Die anderen Enden der Klingen 27 können zugeschärft ausgebildet sein, damit die Klingen 27 beim Einschieben einer erstarrten Probe 38 in diese einschneiden. Die Klingen 27 sind über Schweißnähte 40 am inneren Umfang der Aufnahmehülse 26 gehalten. Beim fortgesetzten Einschieben nehmen die gebogenen Klingen 27 eine gestreckte Gestalt an und winken so als Rückhalteelemente 30. Es versteht sich, dass auch andere Möglichkeiten für die Realisierung der Rückhalteelemente 30 bestehen. Beispielsweise könnten alternativ mehrere kurze dünne klingenartige Rippen, ausgehend vom inneren Umfang der Aufnahmehülse 26 sich radial erstreckend und axial durchgehend vorgesehen sein, die beim Einschieben in den Umfang der erstarrten Probe 38 einschneiden.

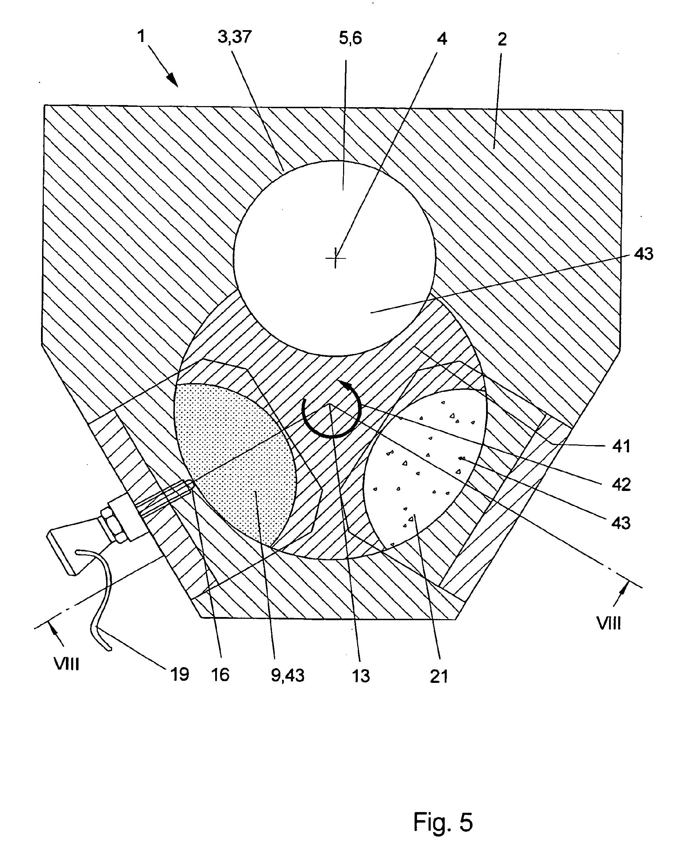

In den Fig. 5 bis 8 ist eine zweite Ausführungsform der Vorrichtung 1 verdeutlicht. Auch diese Vorrichtung 1 weist ein Gehäuse 2 auf, welches einen Rohrleitungsabschnitt 3 besitzt, der an eine Förderleitung für Schokolademasse 6 angeschlossen ist, so dass der Strömungsquerschnitt 5 im Gehäuse 2 von Schokolademasse senkrecht zur Zeichenebene der Fig. 5 durchflossen wird. Der Rohrleitungsabschnitt 3 besitzt die Achse 4.

Der Kolben der Vorrichtung 1 ist hier als Drehkolben 41 ausgebildet, dessen Achse 13 fluchtend und beabstandet zur Achse 4 des Strömungsquerschnitts 5 so angeordnet ist, dass der Strömungsquerschnitt 5 durch den Drehkolben 41 angeschnitten wird. Der Drehkolben 41 wird taktweise gemäß Pfeil 42 in die drei Stellungen gedreht. Der Drehkolben 41 weist in gleicher Teilung mindestens drei Kammern 43 auf. Die Kammern 43 sind identisch ausgebildet und bestehen aus einem Teilabschnitt eines Zylinders, der hier jeweils den Strömungsquerschnitt 5 ergänzt. Die drei Kammern 43 wechseln je nach Drehposition und nehmen die Funktion einer Entnahmekammer, einer Messkammer 9 und einer Aufschmelzkammer 21 ein. Im Bereich der Messkammer 9 ist der Temperaturfühler 16 in die Messkammer 9 einfahrbar bzw. aus dieser herausfahrbar im Gehäuse 2 der Vorrichtung 1 gelagert. Im Bereich der Messkammer 9 besitzt das Gehäuse 2 Kühlkanäle (nicht dargestellt), die von einem Kühlmedium durchströmt werden, so dass die flüssige Schokolademasse im Bereich der Messkammer 9 zur Erstarrung gebracht werden kann. Entsprechend sind im Bereich der Aufschmelzkammer 21 im Gehäuse 2 Kanäle vorgesehen, die an einen Heizkreislauf angeschlossen sind (nicht dargestellt).

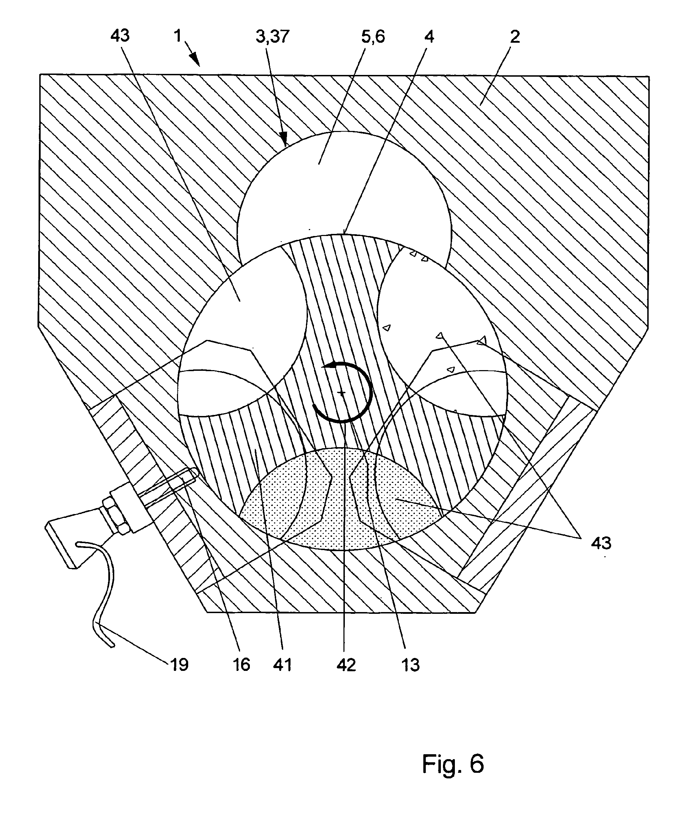

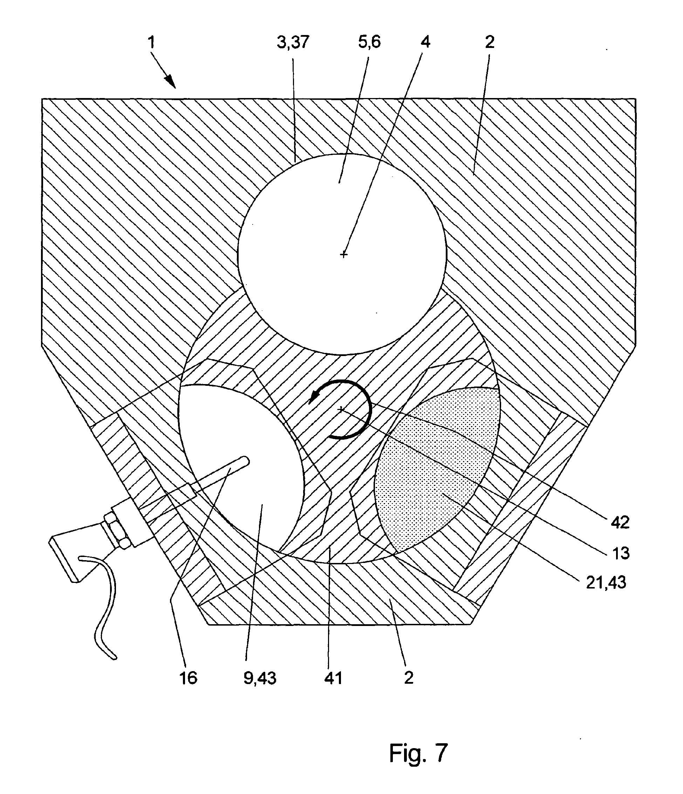

Es ist erkennbar, dass der Temperaturfühler 16 in der Stellung gemäß Fig. 5 sich außerhalb der Messkammer 9 befindet, damit der Drehkolben 41 taktweise fortschreitend angetrieben werden kann. Fig. 6 zeigt die Stellung der Teile nach einer halben Drehung, also in einer Zwischenstellung. Auch hier befindet sich der Temperaturfühler 16 noch außerhalb der Messkammer. Gemäß Fig. 7 wird schließlich die Drehung dargestellt, die nach einer Drehung um eine ganze Teilung mit den Kammern 43 erreicht wird. Die in Fig. 5 obere Kammer 43 hat flüssige Schokolademasse aus dem Strömungsquerschnitt 5 mitgenommen und in einen Bereich überführt, in dem die Kammer 43 die Funktion der Messkammer 9 einnimmt. Der Temperaturfühler 16 wird in die noch flüssige Schokolademasse im Bereich der Messkammer 9 eingefahren (Fig. 7), und durch die einwirkende Kühlung der Schokolademasse findet die Erstarrung statt, wobei die Aufzeichnung der Erstarrungskurve über die Anschlussleitung 19 erfolgt. Gleichzeitig wird die vorangehende Probe, die sich in der vorauseilenden Kammer 43 befindet, in der Aufschmelzkammer 21 aufgeschmolzen und damit in den schmelzflüssigen Zustand überführt. Es ist leicht vorstellbar, dass bei einem weiteren Weiterdrehen des Drehkolbens 41 um eine volle Teilung die aufgeschmolzene Schokolademasse wieder in den Strömungsquerschnitt abgegeben wird. Damit wird zugleich erkennbar, dass der Strömungsquerschnitt 5 völlig frei von jeglichen Hindernissen ist und die

Durchströmung mit der Schokolademasse 6 nicht behindert wird, abgesehen von den Zwischenstellungen gemäß Fig. 6, die jedoch nur sehr kurzzeitig einwirken.

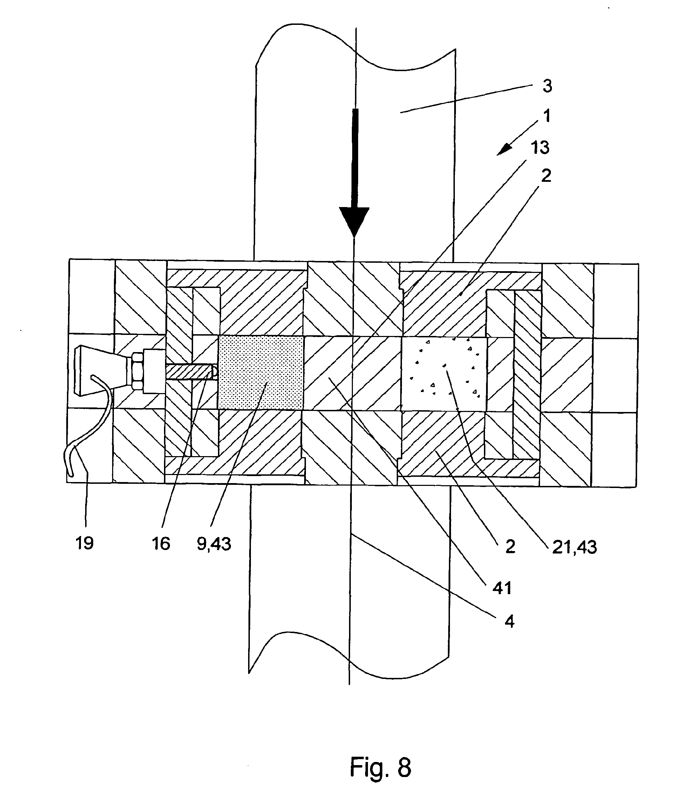

Fig. 8 lässt den Aufbau der Vorrichtung 1 in einem Vertikalschnitt erkennen. Es ist hier nur der Aufbau des Gehäuses und die Anordnung des Drehkolbens 41 im Gehäuse 2 dargestellt. Aus Übersichtlichkeitsgründen ist der Antrieb für den Drehkolben 41 weggelassen, ebenso die Kanäle für ein Kühl- und ein Heizmedium.

### BEZUGSZEICHENLISTE

- 1: Vorrichtung
- 2: Gehäuse
- 3: Rohrleitungsabschnitt
- 4: Achse
- 5: Strömungsquerschnitt
- 6: Schokolademasse
- 7: Pfeil
- 8: Raum
- 9: Messkammer
- 10: Kreislauf

- 11: Kühleinrichtung
- 12: Zylinder
- 13: Achse
- 14: Kolben
- 15: Doppelpfeil
- 16: Temperaturfühler
- 17: Ende
- 18: Kolbenstange
- 19: Anschlussleitung
- 20: Messraum

- 21: Aufschmelzkammer
- 22: Kreislauf
- 23: Heizeinrichtung
- 24: Schmelzraum
- 25: Achse
- 26: Aufnahmehülse
- 27: Klinge
- 28: Kugelventil
- 29: Überströmkanal
- 30: Rückhalteelement

- 31: Einrichtung
- 32: Führungskäfig
- 33: Hülsenabschnitt
- 34: Durchbrechungen
- 35: Achse
- 36: Einrichtung
- 37: Entnahmestelle
- 38: Probe
- 39: Ende
- 40: Schweißnaht

- 41: Drehkolben
- 42: Pfeil
- 43: Kammer

## Patentansprüche

1. Vorrichtung zum Erfassen von Kristallisations-Erstarrungskurven von Schokolade- und ähnlichen Fettmassen, mit
einem Gehäuse (2),
einer darin gebildeten Entnahmestelle (37) für flüssige Schokolade (6),
einer an die Entnahmestelle (37) angeschlossenen und eine gekühlte Wandung aufweisenden Messkammer (9), in der die flüssige Schokolademasse (6) zur Erstarrung gebracht wird,
einem im Gehäuse (2) vorgesehenen bewegbaren Kolben,
einem im Gehäuse (2) vorgesehenen Temperaturfühler (16), und
einer Einrichtung zum Entfernen der erstarrten Probe (38) aus der Messkammer (9),
**dadurch gekennzeichnet, dass**
eine separate Aufschmelzkammer (21) für die erstarrte Probe (38) in Relation zu der Messkammer (9) vorgesehen ist, und
der Hub des Kolbens (14, 41) zum Überführen der erstarrten Probe (38) aus der Messkammer (9) in die Aufschmelzkammer (21) bemessen ist.

2. Vorrichtung nach Anspruch 1, **dadurch gekennzeichnet, dass**
der Kolben als linear hin- und herbeweglicher Kolben (14) ausgebildet ist,
die Aufschmelzkammer (21) fluchtend zur Messkammer (9) angeordnet ist, und
in der Aufschmelzkammer (21) eine Einrichtung (31) zum Festhalten der erstarrten Probe (38) während des Rückhubes des Kolbens (14) vorgesehen ist.

3. Vorrichtung zum Erfassen von Kristallisations-Erstarrungskurven von Schokolade- und ähnlichen Fettmassen, mit
einem Gehäuse (2),
einer darin gebildeten Entnahmestelle (37) für flüssige Schokolade (6),
einer an die Entnahmestelle (37) angeschlossenen und eine gekühlte Wandung aufweisenden Messkammer (9), in der die flüssige Schokolademasse (6) zur Erstarrung gebracht wird,
einem im Gehäuse (2) vorgesehenen bewegbaren Kolben,
einem im Gehäuse (2) vorgesehenen Temperaturfühler (16), und
einer Einrichtung zum Entfernen der erstarrten Probe (38) aus der Messkammer (9),
**dadurch gekennzeichnet, dass**
eine separate Aufschmelzkammer (21) für die erstarrte Probe (38) in Relation zu der Messkammer (9) vorgesehen ist, und
der Kolben als Drehkolben (41) ausgebildet ist und mindestens drei Kammern (43) aufweist, die je nach Drehstellung des Drehkolbens (41) im Gehäuse (2) abwechselnd die Messkammer (9), die Aufschmelzkammer (21) und eine an die Entnahmestelle (37) angeschlossene Kammer bilden.

4. Vorrichtung nach Anspruch 2 oder 3, **dadurch gekennzeichnet, dass** die Entnahmestelle (37) in einem Rohrleitungsabschnitt (3) des Gehäuses (2) angeordnet ist, und dass die Messkammer (9) einerseits und die Aufschmelzkammer (21) andererseits etwa symmetrisch zu der Achse (4) des Rohrleitungsabschnitts (3) gegenüberliegend angeordnet sind.

5. Vorrichtung nach mindestens einem der Ansprüche 1 bis 4, **dadurch gekennzeichnet, dass** die Messkammer (9) und/oder die Aufschmelzkammer (21) etwa zylindrisch oder teilzylindrisch ausgebildet und zumindest im Wesentlichen außerhalb des Strömungsquerschnittes (5) der Entnahmestelle (37) im Gehäuse (2) angeordnet sind.

6. Vorrichtung nach Anspruch 1 und 2, **dadurch gekennzeichnet, dass** im Strömungsquerschnitt (5) der Entnahmestelle (37) im Gehäuse (2) ein insbesondere als durchbrochener Hülsenabschnitt (33) ausgebildeter Führungskäfig (32) für die Überführung der erstarrten Probe (38) aus der Messkammer (9) in die Aufschmelzkammer (21) vorgesehen ist, dessen Achse (35) fluchtend zu den Achsen (13, 25) der Messkammer (9) und der Aufschmelzkammer (21) angeordnet ist.

7. Vorrichtung nach mindestens einem der Ansprüche 1 bis 6, **dadurch gekennzeichnet, dass** im Bereich der Messkammer (9) eine Kühleinrichtung (11) und im Bereich der Aufschmelzkammer (21) eine Heizeinrichtung (23) vorgesehen ist.

8. Vorrichtung nach Anspruch 1 und 2, **dadurch gekennzeichnet, dass** die Einrichtung (31) zum Festhalten der erstarrten Probe (38) während des Rückhubes des Kolbens (14) eine Aufnahmehülse (26) aufweist, in der oder an der als ein Kugelventil (28), gebogene Klingen (27), Widerhaken o. dgl. ausgebildete Rückhalteelemente (30) für die eingeschobene erstarrte Probe (38) vorgesehen sind.

9. Vorrichtung nach Anspruch 3, **dadurch gekennzeichnet, dass** der Drehkolben (41) mit seiner Achse (13) parallel und beabstandet zu der Achse (4) der Entnahmestelle (37) im Gehäuse (2) angeordnet ist.

10. Vorrichtung nach Anspruch 3 und 9, **dadurch gekennzeichnet, dass** der im Gehäuse (2) vorgesehene Temperaturfühler (16) des Drehkolbens (41) in die Messkammer (9) ein- und ausfahrbar gelagert ist.

## Claims

1. An apparatus for determining crystallization solidification curves of chocolate masses and other fat masses, comprising
a housing (2),
a removal location (37) for liquid chocolate (6), the removal location (37) being formed in the housing (2),
a measurement chamber (9) in which the liquid chocolate mass (6) is caused to solidify, the measurement chamber (9) being connected to the removal location (37) and including a cooled wall,
a movable piston being provided in the housing (2),
a temperature sensor (16) being provided in the housing (2), and
a unit for removing the solidified sample (38) from the measurement chamber (9), **characterised in that**
a separate melting chamber (21) for the solidified sample (38) is provided in relation to the measurement chamber (9), and
the stroke of the piston (14, 41) is dimensioned to transfer the solidified sample (38) from the measurement chamber (9) into the melting chamber (21).

2. The apparatus of claim 1, **characterised in that**
the piston is designed as a piston (14) oscillating back and forth in a linear direction,
the melting chamber (21) is arranged to be aligned to the measurement chamber (9), and
a unit (31) for retaining the solidified sample (38) during the backward stroke of the piston (14) is provided in the melting chamber (21).

3. An apparatus for determining crystallization solidification curves of chocolate masses and other fat masses, comprising
a housing (2),
a removal location (37) for liquid chocolate (6), the removal location (37) being formed in the housing (2),
a measurement chamber (9) in which the liquid chocolate mass (6) is caused to solidify, the measurement chamber (9) being connected to the removal location (37) and including a cooled wall,
a movable piston being provided in the housing (2),
a temperature sensor (16) being provided in the housing (2), and
a unit for removing the solidified sample (38) from the measurement chamber (9), **characterised in that**
a separate melting chamber (21) for the solidified sample (38) is provided in relation to the measurement chamber (9), and
the piston is designed as a rotary piston (41) and includes at least three chambers (43) alternately forming the measurement chamber (9), the melting chamber (21) and a chamber being connected to the removal location (37) depending on the rotational position of the rotary piston (41) in the housing (2).

4. The apparatus of claim 2 or 3, **characterised in that** the removal location (37) is arranged in a tube section (3) of the housing (2) and that the measurement chamber (9) and the melting chamber (21) are arranged at opposite sides and approximately symmetric to the axis (4) of the tube section (3).

5. The apparatus of at least one of claims 1 to 4, **characterised in that** the measurement chamber (9) and/or the melting chamber (21) are designed to be approximately cylindrical or at least partly cylindrical and that they are at least substantially arranged in the housing (2) outside of the cross-section of the flow (5) of the removal location (37).

6. The apparatus of claim 1 and 2, **characterised in that** a guiding cage (32) is provided in the housing (2) in the cross-section of the flow (5), the guiding cage (32) especially being designed as a bush section (33) including openings, the guiding cage (32) serving to transfer the solidified sample (38) from the measurement chamber (9) into the melting chamber (21), the axis (35) of the guiding cage (32) being arranged to be aligned to the axes (13, 25) of the measurement chamber (9) and of the melting chamber (21).

7. The apparatus of at least one of claims 1 to 6, **characterised in that** a cooling unit (11) is provided in the region of the measurement chamber (9) and a heating unit (23) is provided in the region of the melting chamber (21).

8. The apparatus of claim 1 and 2, **characterised in that** the unit (31) for retaining the solidified sample (38) during the backward stroke of the piston (14) includes a receiving bush (26) in which or at which retaining elements (30) for the inserted solidified sample (38) are provided, the retaining elements (30) being designed as a ball valve (28), bent knives (27), barbed elements and the like.

9. The apparatus of claim 3, **characterised in that** the rotary piston (41) with its axis (13) is arranged in the housing (2) to be parallel to and spaced apart from the axis (4) of the removal location (37).

10. The apparatus of claim 3 and 9, **characterised in that** the temperature sensor (16) of the rotary piston (41) being provided in the housing (2) is supported to be movable into the measurement chamber (9) and out off the measurement chamber (9).

## Revendications

1. Appareil pour déterminer des courbes de solidification par cristallisation de masses de chocolat et de masses de graisse similaires, avec
un boîtier (2),
un emplacement de prélèvement (37) de chocolat liquide (6) qui est formé dans le boîtier,
une chambre de mesure (9) raccordée à l'emplacement de prélèvement (37) et présentant une paroi refroidie, dans laquelle chambre la masse de chocolat liquide (6) est amenée à se solidifier,
un piston mobile prévu dans le boîtier (2),
une sonde de température (16) prévue dans le boîtier (2), et
un dispositif pour enlever de la chambre de mesure (9) l'échantillon solidifié (38), **caractérisé en ce**
**qu'**une chambre de fusion (21) séparée pour l'échantillon solidifié (38) est prévue en relation avec la chambre de mesure (9), et
la course du piston (14, 41) est dimensionnée pour le transfert de l'échantillon solidifié (38) à partir de la chambre de mesure (9) vers la chambre de fusion (21).

2. Appareil selon la revendication 1, **caractérisé en ce que**
le piston est réalisé en tant que piston (14) pouvant effectuer un mouvement de va-et-vient linéaire,
la chambre de fusion (21) est disposée en affleurement avec la chambre de mesure (9), et
dans la chambre de fusion (21), il est prévu un dispositif (31) pour le maintien de l'échantillon solidifié (38) pendant la course de retour du piston (14).

3. Appareil pour déterminer des courbes de solidification par cristallisation de masses de chocolat et de masses de graisse similaires, avec
un boîtier (2),
un emplacement de prélèvement (37) de chocolat liquide (6) qui est formé dans le boîtier,
une chambre de mesure (9) raccordée à l'emplacement de prélèvement (37) et présentant une paroi refroidie, dans laquelle chambre la masse de chocolat liquide (6) est amenée à se solidifier,
un piston mobile prévu dans le boîtier (2),
une sonde de température (16) prévue dans le boîtier (2), et
un dispositif pour enlever de la chambre de mesure (9) l'échantillon solidifié (38), **caractérisé en ce**
**qu'**une chambre de fusion (21) séparée pour l'échantillon solidifié (38) est prévue en relation avec la chambre de mesure (9), et
le piston est réalisé en tant que piston rotatif (41) et présente au moins trois chambres (43) qui, en fonction de la position de rotation respective du piston rotatif (41) dans le boîtier (2), forment alternativement la chambre de mesure (9), la chambre de fusion (21) et une chambre raccordée à l'emplacement de prélèvement (37).

4. Appareil selon la revendication 2 ou 3, **caractérisé en ce que** l'emplacement de prélèvement (37) est disposé dans un tronçon de conduite tubulaire (3) du boîtier (2), et **en ce que** la chambre de mesure (9) d'une part et la chambre de fusion (21) d'autre part sont disposées de façon opposée à peu près symétriquement par rapport à l'axe (4) du tronçon de conduite tubulaire (3).

5. Appareil selon au moins une des revendications 1 à 4, **caractérisé en ce que** la chambre de mesure (9) et/ou la chambre de fusion (21) sont réalisées de façon approximativement cylindrique ou partiellement cylindrique, et **en ce qu'**elles sont disposées au moins pour l'essentiel à l'extérieur de la section transversale d'écoulement (5) de l'emplacement de prélèvement (37) dans le boîtier (2).

6. Appareil selon les revendications 1 et 2, **caractérisé en ce que**, dans la section transversale d'écoulement (5) de l'emplacement de prélèvement (37) dans le boîtier (2), il est prévu une cage de guidage (32), réalisée en particulier en tant que tronçon de manchon (33) percé, pour le transfert de l'échantillon solidifié (38) à partir de la chambre de mesure (9) vers la chambre de fusion (21), cage dont l'axe (35) est disposé en affleurement avec les axes (13, 25) de la chambre de mesure (9) et de la chambre de fusion (21).

7. Appareil selon au moins une des revendications 1 à 6, **caractérisé en ce qu'**il est prévu un dispositif de refroidissement (11) dans la zone de la chambre de mesure (9) et un dispositif de chauffage (23) dans la zone de la chambre de fusion (21).

8. Appareil selon les revendications 1 et 2, **caractérisé en ce que** le dispositif (31) pour le maintien de l'échantillon solidifié (38) pendant la course de retour du piston (14) présente un manchon de réception (26) dans lequel ou sur lequel il est prévu, pour l'échantillon solidifié (38) inséré, des éléments de retenue (30) réalisés en tant que clapet à bille (28), lames courbes (27), ardillons ou similaires.

9. Appareil selon la revendication 3, **caractérisé en ce que** le piston rotatif (41) avec son axe (13) est disposé parallèlement à, et à distance de, l'axe (4) de l'emplacement de prélèvement (37) dans le boîtier.

10. Appareil selon les revendications 3 et 9, **caractérisé en ce que** la sonde de température (16) du piston rotatif (41) prévue dans le boîtier (2) est supportée de façon à pouvoir être introduite dans la chambre de mesure (9) et sortie de la chambre de mesure (9).
